# EUROPEAN PATENT APPLICATION

(11) **EP 1 358 890 A1**
(43) Date of publication of application: **05.11.2003**
(21) Application number: 02076757.0
(22) Date of filing: 03.05.2002
(51) Int. Cl.: A61K 51/08

(54) **Benzothienyl analogue of somatostatine, selective for certain somatostatin receptors**

(71) Applicant: BioSynthema, Inc, Saint Louis, MO 63108 (US)
(72) Inventor: De Jong, Marion, Ph.D., 3137 TN Vlaardingen (NL); Mäcke, Helmut Robert, Prof., 4031 Lörrach (DE); Ginj, Mihaela, Ph.D., 4058 Basel (CH); Krenning, Eric Paul, Prof., 3062 JC Rotterdam (NL); Reubi, Jean Claude, Prof., 3084 Wabern (CH)
(74) Representative: Hooiveld, Arjen Jan Winfried

(57) **Abstract**

The invention relates to a peptide compound having an improved binding affinity to somatostatin receptors, comprising a peptide and a chelating group covalently linked to a free amino group of said peptide , wherein said peptide is or comprises a somatostatin analogue carrying a 3-benzothienylalanine residue in its 3-position.

The invention further relates to said peptide compound labelled wih a detectable element or with a therapeutic radionuclide, as well as to a diagnostic method and to a method for the therapeutic treatment of tumors, by using the labelled compounds.

## Description

The invention relates to a peptide compound having an improved binding affinity to somatostatin receptors, comprising a peptide and a chelating group covalently linked to a free amino group of said peptide.

Such peptide compounds and their radiolabelled derivatives can be used for therapy of somatostatin - receptor positive tumors. Detectably labelled somatostatin - peptide compounds are also useful for in vivo imaging. See in this respect the patent publications of Albert et al. (US 5,753,627; US 5,776,894), of Krenning et al. (US 6,123,916), of De Jong et al. (WO 00/18440), of Srinivasan et al. (US 5,804,157; 5,830,431), and recent international patent application PCT/EP01/04764. Albert et al. disclose complexed somatostatin peptides for in vivo imaging of somatostatin receptor - positive tumors, which peptides are derived from somatostatin analogues, carrying an optionally substituted phenylalanine residue or a beta- or 2-naphthylalanine residue in its 3-position. Selective internal tumor therapy with radiolabelled peptides has become very important in nuclear medicine in the past years. Especially somatostatin derivatives have been successfully applied in the clinic for tumor diagnosis and therapy, showing that the principle of receptor targeting is working in practice. For more than four years already much experience has been gained in clinical trials with the use of ⁹⁰Y - labelled DOTA-[Tyr³]-octreotide (DOTA-TOC) for tumor therapy (M. de Jong: Eur. J. Nucl. Med. **26**, 1999, 693-698).

Yet DOTA-TOC only shows high affinity to the somatostatin receptor subtype 2 (sst 2), whereas the affinity to other somatostatin subtypes, in particular sst 3 and sst 5, which are found also in a variety of tumors, is too low to contribute essentially to tumor targeting. For example, most thyroid tumors express these last-mentioned somatostatin receptor subtypes, but carry sst 2 only on a low level ( E.B. Forssell-Aronsson et al.: J. Nucl. Med. **41**, 2000, 636-642).

Recently, additional results of labelled octreotide analogues have been disclosed: J. Labelled Cpd. Radiopharm. **44,** Suppl. 1 (2001), 5697-5699 (see also: Eur. J. Nucl. Med. **28/8,** OS 24 (2001), 966). In this paper it is suggested, that modification of the 3-position of octreotide, especially with hydrophobic aromatic amino acids, could improve the sst 3 and sst 5 receptor affinities. It has indeed be found, according to this publication, that DOTA-[1-Nal³]-octreotide (DOTA-NOC), i.e. an octreotide analogue having in its 3-position 1-naphyhylalanine substituted for 1-phenylalanine, has a significant affinity to sst 3 and sst 5 somatostatin receptors, attended by a superior sst 2 receptor affinity.

Still there remains a need for peptide compounds having a further improved binding affinity to a plurality of somatostatin receptors.

It is therefore the objective of the present invention to provide a peptide compound which has an improved binding affinity to a plurality of somatostatin receptor subtypes, compared with the above known somatostatin peptides. On account of its multispecificity, such a peptide compound , in particular after labelling with a suitable radionuclide, could be therapeutically used to more effectively treat a broad variety of tumors. In addition, after labelling with a suitable detectable element, such a peptide compound should have an improved suitability for in vivo detecting and localizing tissues, in particular tumors and metastases thereof, carrying somatostatin receptor types in varying levels.

This objective can be achieved, according to the present invention, by a peptide compound as defined in the opening paragraph, wherein said peptide is or comprises a somatostatin analogue carrying a 3-benzothienylalanine residue in its 3-position.

It has been found, that the new peptide compounds of the invention have an unexpectedly high affinity to a plurality of somatostatin receptor subtypes. These favourable results are against expectation, because the hydrophobic character of the peptide is not improved by substituting phenylalanine or 1-naphthylalanine by 3-benzothienylalanine. This favourable binding affinity makes the new peptide compounds promising candidates both for diagnosis, after labelling, and for tumor therapy.

More in particular, the present invention relates to a new peptide compound as defined above, wherein the peptide has the general formula

H-(A⁰)ₙ-(D)A¹*-cyclo*[Cys²-A³-A⁴-A⁵-A⁶-Cys⁷]-A⁸ (I)

wherein:
n is 0 or 1,
A⁰ is optionally halogenated Tyr or Phe, or wherein
A⁰ is a trifunctional amino acid residue to which an additional peptide part, comprising 3 to 12 amino acid residues which form at least once the Arg-Gly-Asp sequence, is covalently linked,
A¹ is optionally halogenated Tyr, or optionally halogenated or methylated Phe or Nal,
A³ is 3-benzothienylalanyl,
A⁴ is Trp, optionally N-methylated in its side-chain,
A⁵ is Lys, optionally N-methylated in its side-chain,
A⁶ is Thr, Val, Ser, Phe or Ile, and
A⁸ is Thr, Trp or Nal, wherein the terminal carboxy group may be modified to an alcohol or an , optionally C1-C3 alkylated, amide group.

Suitable examples of the above new peptides of formula I are:
(1) H-(D)Phe-*cyclo*[Cys-3-benzothienylalanyl-(D)Trp-Lys-Thr-Cys]-Thr-ol
(2) H-(D)Nal-*cyclo*[Cys-3-benzothienylalanyl-(D)Trp-Lys-Val-Cys]-Thr-NH₂

The above examples are covered by the general formula II, encompassing preferred peptides according to the invention.

H-(Á⁰)ₙ-(D)Á¹-*cyclo*[Cys²-A³-Trp⁴-Lys⁵-Á⁶-Cys⁷]-Á⁸ (II)

wherein:
n is 0 or 1,
Á⁰ is optionally halogenated Tyr,
Á¹ is optionally halogenated Tyr, or Phe or Nal,
A³ is 3-benzothienylalanyl,
Á⁶ is Thr or Val, and
Á⁸ is Thr-ol, Thr-OH or Thr-NH₂.

The peptide compound according to the invention comprises a chelating group covalently linked to the N-terminal free amino group of the peptide. Various well-known chelating groups can be used, for example, those selected from:
(i) N₂S₂-, N₃S- and N₄-tetradentate ring structure containing groups, (ii) isocyanate, carbonyl, formyl, diazonium, isothiocyanate and alkoxycarbimidoyl containing groups, (iii) groups derived from N-containing di- and polyacetic acids and their derivatives, and from (iv) 2-iminothiolane and 2-iminothiacyclohexane containing groups.
The chelating groups sub (iii) above are preferred, encompassing chelating groups derived from ethylenediamine tetraacetic acid (EDTA), diethylenetriamine pentaacetic acid (DTPA), ethyleneglycol-OO'-bis(2-aminoethyl)-N,N,N',N'-tetraacetic acid (EGTA), N,N'-bis(hydroxybenzyl)ethylenediamine-N,N'-diacetic acid (HBED), triethylenetetramine hexaacetic acid (TTHA), substituted EDTA or DTPA, 1,4,7,10-tetraazacyclododecane-N,N',N",N"'-tetraacetic acid (DOTA), 1,4,7-triazacyclonane-1,4,7-triacetic acid (NOTA) or 1,4,8,11-tetraazacyclotetradecane-N,N',N",N"'-tetraacetic acid (TETA).
The method of linking the chelating group to a free amino group of the peptide for obtaining the peptide compound of the invention is generally known in the art. The synthesis of peptide compounds having the above chelating group sub (iv) is described in WO 89/07456. Peptide compounds wherein the peptide comprises a somatostatin analogue, more in particular wherein the peptide has the above general formula I, wherein n is 1 and A° is a trifunctional amino acid residue to which an additional peptide part, comprising 3 to 12 amino acid residues which form at least once the Arg-Gly-Asp sequence, is covalently linked, can be prepared according to the above PCT/EP01/04764.

Generally, for the purpose in view, the peptide compound of the invention is labelled with a detectable element selected from gamma- or positron-emitting radionuclides, Auger-electron-emitting isotopes and paramagnetic ions of nonradioactive elements, or with a therapeutic radionuclide.
Suitable detectable elements for imaging purposes are gamma- or positron-emitting radionuclides, selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ⁵¹Cr, ²⁴Na, ¹⁵⁷Gd, ^{52m}Mn, ¹⁶²Dy, ¹²³I, ¹³¹I, ⁷⁵Br and ⁷⁶Br, or paramagnetic ions of elements, selected from the group consisting of nonradioactive Gd, Fe, Mn and Cr.
In addition to the use of radioisotopes as detectable elements, which enables in vivo detection by a gamma camera, paramagnetic ions of nonradioactive elements, such as Gd, Fe, Mn or Cr, preferably of Gd, can be used, viz. for in vivo detection by MRI.

For therapeutic purposes the compounds of the invention can advantageously be labelled with therapeutic radionuclides, selected from the group consisting of ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ²¹¹At, ¹¹¹In and ¹⁵³Sm.

Chelation of the above metal isotopes can easily be effected in a manner known per se for related compounds, for example, by bringing the peptide compound of the invention in contact with a compound, often a salt, of the desired isotope in a suitable solvent or diluent, if desired at higher temperature.
The preparation of radioactive-halogen labelled peptide compounds according to the present invention can be carried out by a method as described for related compounds in the above-mentioned WO 00/18440, in order to introduce the desired halogen radionuclide into an aromatic nucleus in position 0 or 1 of the peptide. This labelling can conveniently be performed by introducing a halogen atom or a radioactive halogen atom into an radioactive nucleus, preferably an activated aromatic nucleus such as tyrosyl, present in the above position in the peptide compound, if necessary followed by exchange with the desired halogen radionuclide.
The radiohalogenation reaction is preferably performed by reacting the peptide compound with a solution of an alkali metal radionuclide selected from ¹²³I⁻, ¹³¹I⁻, ²¹¹At⁻, ⁷⁵Br⁻ and ⁷⁶Br⁻ under the influence of a halide-oxidizing agent, such as chloramine T or iodogen. Alternatively, the above substitution reaction can be carried out with nonradioactive halogen, after which halo-exchange with radioactve halogen is performed, e.g. as described in European patent 165630.

The present invention further relates to a pharmaceutical composition, comprising in addition to a pharmaceutically acceptable carrier and, if desired , at least one pharmaceutically acceptable adjuvant, as the active substance a labelled peptide compound as defined above, or a pharmaceutically acceptable salt thereof.
Such pharmaceutical compositions can be used for diagnostic purposes; then the peptide compounds are provided with detectable elements as described above. If the compositions are intended for tumor therapy, advantageously the above-mentioned therapeutic radionuclides can be used for labelling the peptide compounds.

The present invention also relates to a method for detecting and localizing tissues having somatostatin receptors in the body of a warm-blooded living being, This diagnostic method comprises (i) administering to said being a pharmaceutical composition, labelled with a suitable detectable element, as defined above, comprising the active substance in a quantity sufficient for external imaging, and thereupon (ii) subjecting said being to external imaging to determine the targeted sites in the body of said being in relation to the background activity, in order to allow detection and localisation of said tissues in said body.
The present invention further relates to a method for the therapeutic treatment of tumors, having on their surface somatostatin receptors, in the body of a warm-blooded living being, which comprises administering to said being a pharmaceutical composition labelled with a suitable therapeutic radionuclide, as define above, comprising the active substance in a quantity effective for combating or controlling tumors.

It is sometimes hardly possible to put the ready-for use radiolabelled composition at the disposal of the user, in connection with the often poor shelf life of the radiolabelled peptide compound and/or the short half-life of the radionuclide used. In such cases the user him- or herself can carry out the labelling reaction with the radionuclide in the clinical hospital or laboratory. For this purpose the various reaction ingredients are then offered to the user in the form of a so-called "kit". It will be obvious that the manipulations necessary to perform the desired reaction should be as simple as possible to enable the user to prepare from the kit the radioactive-labelled composition by using the facilities that are at his disposal. Therefore the invention also relates to a kit for preparing a radiopharmaceutical composition.
Such a kit according to the present invention may conveniently comprise a peptide compound as defined hereinbefore, viz. derived from a peptide that is or comprises a somatostatin analogue carrying a 3-benzothienylalanine residue in its 3-position, to which substance, if desired, an inert pharmaceutically acceptable carrier and/or formulating agents and/or adjuvants are added, (ii) a solution of a radionuclide compound selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ⁵¹Cr, ²⁴Na, ¹⁵⁷Gd, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh and ¹⁵³Sm, and (iii) instructions for use with a prescription for reacting the ingredients present in the kit. The kit according to the invention preferably comprises a peptide compound derived from a peptide having the general formula I, wherein the symbols have the meanings given hereinbefore.

The invention will now be described in greater detail with reference to the following specific Examples.

### EXAMPLE I

### Synthesis of peptide compounds

The new peptide compounds or peptide conjugates of the invention are synthesized by Fmoc-solid-phase synthesis on 2-chloro-tritylchloride resin (Int. J. Pept. Protein Res. 35, 1990, 161-214). According to this method Fmoc-protected amino-acids are successively coupled, each time followed by cleavage of the protecting Fmoc- group in basic medium. Finally cleavage of the fully protected conjugates from the resin, oxidative cyclization to the cystin-containing cyclic peptide, and introduction of the DOTA-chelator, (e.g. as described by Heppeler et al. in Chem Eur. J. 1999; 5: 1974-1981), leads to the desired peptide compounds comprising the peptides (1) and (2), mentioned hereinbefore, carrying DOTA as the metal-chelating moiety, linked to the free amino group of the peptide.

### EXAMPLE II

### Labelling of peptide compounds

The above DOTA carrying peptide compound (1) is labelled with ¹¹¹In by dissolving the compound in 0.01 M acetic acid, mixing this solution with ¹¹¹InCl₃ -solution (1 mCi / 100µl) in 0.05 M aqueous sodium acetate at higher temperature, and finally neutralizing the solution with HEPES buffer.
Labelling with ⁹⁰Y, obtained from a ⁹⁰Sr - ⁹⁰Y radionuclide generator, is performed as follows. A solution of the above DOTA carrying peptide compound (1) in 0.01 M acetic acid is treated with ⁹⁰Y (1.0 mCi / 50 µl 0.5 M acetate solution). The mixture is left for approx. 1 hr at higher temp. to effect chelation.

### EXAMPLE III

### In vitro binding experiments

In vitro binding affinities are determined using transfected cell lines with somatostatin human receptor subtypes (hsst) 2, 3 and 5, as described by Reubi et al. in Eur. J. Nucl. Med. **27**, 2000, 273-282. The affinity profiles (IC₅₀ values), determined for these somatostatin receptor subtypes, are presented in Table A below. In this table the results of a labelled peptide compound according to the invention, viz. ⁹⁰Y-labelled DOTA-(D)Phe-*cyclo*[Cys-(D)3-benzothienylalanyl-(D)Trp-Lys-Thr-Cys]-Thr-ol (Y-DOTA-[BzThi³]-OC; cpd. **7**), are compared with those of Y-DOTA-[1-Nal³]-OC (cpd. 8), referenced to the respective data of somatostatin 28 (cpd. 0).

For purpose of comparison, recently published results (Reubi et al. - see above) of an additional series of IC₅₀ values are also presented in Table A: OC to Y-DOTA-TOC (cpds. 3-6), also referred to the corresponding data of somatostatin S28 (SS-28; cpd. 0).

**Table A**

| Affinity profiles (IC₅₀) for human sst (hsst) 2, 3 and 5 receptors. All values are IC₅₀±SEM in nM. The number of experiments is given in parentheses. OC = Octreotide = H-(D)Phe¹-*cyclo*[Cys²-Phe³-(D)Trp⁴-Lys⁵-Thr⁶-Cys⁷]-Thr⁸-ol LAN = Lanreotide = H-(D)2-Nal-*cyclo*[Cys-Phe-(D)Trp-Lys-Val-Cys]-Thr-NH₂ TOC = H-(D)Phe*-cyclo*[Cys-Tyr-(D)Trp-Lys-Thr-Cys]-Thr-ol | | | | |
|---|---|---|---|---|
| cpd. no. | compound | hsst 2 | hsst 3 | hsst 5 |
| 0 | SS-28 | 2.7±0.3 (19) | 7.7±0.9 (15) | 4.0±0.3 (19) |
| 3 | OC | 2.0±0.7 (5) | 187±55 (3) | 22±6 (5) |
| 4 | Y-DOTA-OC | 20±2 (5) | 27±8 (5) | 57±22 (4) |
| 5 | Y-DOTA-LAN | 23±5 (4) | 290±105 (4) | 16±3.4 (4) |
| 6 | Y-DOTA-TOC | 11±1.7 (6) | 389±135 (5) | 114±29 (5) |
| 0 | SS-28 | 2.7±0.3 (8) | 3.7±0.3 (8) | 2.9±0.4 (8) |
| 7 | Y-DOTA-[BzThi³]-OC | 3.4 | 13 | 4.1 |
| 8 | Y-DOTA-[1-Nal³]-OC | 3.3±0.2 (3) | 26±1.9 (3) | 10±1.6 (3) |

The above results show that a peptide compound according to the present invention (cpd. 7) has a highly promising affinity profile with respect to somatostatin receptors. It is binding significantly better to sst 5 than both compounds 5 and 8, and is much better than compounds 4 and 6 on this receptor, even taken into account the different values for the SS-28 (cpd. 0) references. The affinity of cpd. 7 to sst 3 is also significantly better than for compounds 4 and 8, and even approx. ten times better than for cpd. 5. Surprising is the affinity to the important receptor sst 2. Both compounds 7 and 8 have an approx. three times better binding affinity to sst 2 than even cpd. 6.

## Claims

1. A peptide compound having an improved binding affinity to somatostatin receptors, comprising a peptide and a chelating group covalently linked to a free amino group of said peptide , wherein said peptide is or comprises a somatostatin analogue carrying a 3-benzothienylalanine residue in its 3-position.

2. A peptide compound as claimed in claim 1, wherein the peptide has the general formula
H-(A⁰)ₙ-(D)A¹-*cyclo*[Cys²-A³-A⁴-A⁵-A⁶-Cys⁷]-A⁸ (I)
wherein:
n is 0 or 1,
A⁰ is optionally halogenated Tyr or Phe, or wherein
A⁰ is a trifunctional amino acid residue to which an additional peptide part, comprising 3 to 12 amino acid residues which form at least once the Arg-Gly-Asp sequence, is covalently linked,
A¹ is optionally halogenated Tyr, or optionally halogenated or methylated Phe or Nal,
A³ is 3-benzothienylalanyl,
A⁴ is Trp, optionally N-methylated in its side-chain,
A⁵ is Lys, optionally N-methylated in its side-chain,
A⁶ is Thr, Val, Ser, Phe or Ile, and
A⁸ is Thr, Trp or Nal, wherein the terminal carboxy group may be modified to an alcohol or an , optionally C1-C3 alkylated, amide group.

3. A peptide compound as claimed in claim 2, wherein the peptide is a somatostatin analogue of the general formula
H-(Á⁰)ₙ-(D)Á¹-*cyclo*[Cys²-A³-Trp⁴-Lys⁵-Á⁶-Cys⁷]-Á⁸ (II)
wherein:
n is 0 or 1,
Á⁰ is optionally halogenated Tyr,
Á¹ is optionally halogenated Tyr, or Phe or Nal,
A³ is 3-benzothienylalanyl,
Á⁶ is Thr or Val, and
Á⁸ is Thr-ol, Thr-OH or Thr-NH₂.

4. A peptide compound as claimed in any of the preceding claims, wherein the chelating group is selected from: (i) N₂S₂-, N₃S- and N₄-tetradentate ring structure containing groups, (ii) isocyanate, carbonyl, formyl, diazonium, isothiocyanate and alkoxycarbimidoyl containing groups, (iii) groups derived from N-containing di- and polyacetic acids and their derivatives, and from (iv) 2-iminothiolane and 2-iminothiacyclohexane containing groups.

5. A peptide compound as claimed in claim 4, wherein the chelating group is derived from ethylenediamine tetraacetic acid (EDTA), diethylenetriamine pentaacetic acid (DTPA), ethyleneglycol-OO'-bis(2-aminoethyl)-N,N,N',N'-tetraacetic acid (EGTA), N,N'-bis(hydroxybenzyl)ethylenediamine-N,N'-diacetic acid (HBED), triethylenetetramine hexaacetic acid (TTHA), substituted EDTA or DTPA, 1,4,7,10-tetraazacyclododecane-N,N',N",N"'-tetraacetic acid (DOTA), 1,4,7-triazacyclonane-1,4,7-triacetic acid (NOTA) or 1,4,8,11-tetraazacyclotetradecane-N,N',N'',N'''-tetraacetic acid (TETA).

6. A peptide compound as claimed in any of the preceding claims, which compound is labelled with a detectable element selected from gamma- or positron-emitting radionuclides, Auger-electron-emitting isotopes and paramagnetic ions of nonradioactive elements, or with a therapeutic radionuclide.

7. A peptide compound as claimed in claim 6, labelled with a gamma- or positron-emitting radionuclide, selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ⁵¹Cr, ²⁴Na, ¹⁵⁷Gd, ^{52m}Mn, ¹⁶²Dy, ¹²³I, ¹³¹I, ⁷⁵Br and ⁷⁶Br, or with a paramagnetic ion of an element, selected from the group consisting of nonradioactive Gd, Fe, Mn and Cr.

8. A peptide compound as claimed in claim 6, labelled with a therapeutic radionuclide, selected from the group consisting of ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123m}Rh, ²¹¹At, ¹¹¹In and ¹⁵³Sm.

9. A pharmaceutical composition comprising in addition to a pharmaceutically acceptable carrier and, if desired , at least one pharmaceutically acceptable adjuvant, as the active substance a labelled peptide compound as claimed in claim 6, or a pharmaceutically acceptable salt thereof.

10. A composition as claimed in claim 9, wherein the peptide compound is labelled with a gamma- or positron-emitting radionuclide, or with a paramagnetic ion of a nonradioactive element, as defined in claim 7.

11. A composition as claimed in claim 9, wherein the peptide compound is labelled with a therapeutic radionuclide as defined in claim 8.

12. A method for detecting and localizing tissues having somatostatin receptors in the body of a warm-blooded living being, which comprises (i) administering to said being a composition as claimed in claim 10, comprising the active substance in a quantity sufficient for external imaging, and thereupon (ii) subjecting said being to external imaging to determine the targeted sites in the body of said being in relation to the background activity, in order to allow detection and localisation of said tissues in said body.

13. A method for the therapeutic treatment of tumors having on their surface somatostatin receptors in the body of a warm-blooded living being, which comprises administering to said being a composition as claimed in claim 11, comprising the active substance in a quantity effective for combating or controlling tumors.

14. A kit for preparing a pharmaceutical composition as claimed in claim 9, comprising (i) a peptide compound as claimed in claim 1, to which substance, if desired, an inert pharmaceutically acceptable carrier and/or formulating agents and/or adjuvants are added, (ii) a solution of a radionuclide compound, said radionuclide being selected from the group consisting of ^{99m}Tc, ²⁰³Pb, ⁶⁶Ga, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ⁵¹Cr, ²⁴Na, ¹⁵⁷Gd, ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁰⁹Pd, ¹⁶⁵Dy, ¹⁷⁷Lu, ¹⁶¹Tb, ^{123mRh and 53}Sm, and (iii) instructions for use with a prescription for reacting the ingredients present in the kit.

15. A kit as claimed in claim 14, wherein the peptide compound comprises a peptide as defined in claim 2.
